(19) Europäisches Patentamt
European Patent Office
Office européen des brevets

(11) Publication number: 0 274 909 B1

# (12) EUROPEAN PATENT SPECIFICATION

(45) Date of publication of patent specification: 10.08.94

(51) Int. Cl.[5]: C07B 41/00, C07C 27/12, C07C 29/50, C07C 45/33, B01J 31/18, C07F 11/00, C07F 13/00, C07D 487/22, //C07C49/08,C07C49/403, C07C35/08,C07C31/02

(21) Application number: 87311480.5

(22) Date of filing: 29.12.87

## (54) Hydrocarbon oxidations catalyzed by azide- or nitride-activated metal coordination complexes.

(30) Priority: 02.01.87 US 246
02.01.87 US 247

(43) Date of publication of application:
20.07.88 Bulletin 88/29

(45) Publication of the grant of the patent:
10.08.94 Bulletin 94/32

(84) Designated Contracting States:
BE DE FR GB IT NL

(56) References cited:
EP-A- 0 079 705

JOURNAL OF THE CHEMICAL SOCIETY, CHEMICAL COMMUNICATIONS, no. 6, 1983, pages253-254; D. MANSUY et al.: "Mono-oxygenase-like dioxygen activation leading toalkane hydroxylation and olefin epoxidation by an MnIII(porphyrin)-ascorbate

biphasic system"

(73) Proprietor: Sun Company, Inc. (R&M)
1801 Market Street
Philadelphia, PA 19103-1699 (US)

(72) Inventor: Ellis, Paul E.
1179 Glenside Avenue
Downington, PA 19335 (US)
Inventor: Lyons, James E.
211 Cooper Drive
Wallingford, PA 19086 (US)
Inventor: Myers, Harry K.
Limestone Road
Cochranville, PA 19330 (US)

(74) Representative: Lewin, John Harvey et al
Elkington and Fife
Prospect House
8 Pembroke Road
Sevenoaks, Kent TN13 1XR (GB)

EP 0 274 909 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**JOURNAL OF THE AMERICAN CHEMICAL SOCIETY, vol. 102, no. 20, 24th September1980, pages 6374-6375, American Chemical Society, US; C.L. HILL et al.: "Alkaneactivation and functionalization under mild conditions by a homogeneousmanganese(III) porphyrin-iodosylbenzene oxidizing systems"**

## Description

### Field of the Invention

This invention relates to a novel process for the oxidation of hydrocarbons. More particularly, this invention relates to the catalytic oxidation of a wide range of oxidizable hydrocarbons, particularly alkanes, with air or oxygen. The catalyst is a ligand complex of transition metals activated by an azide or nitride group bonded to the metal. Novel classes of the azide catalysts are also claimed herein.

### Background of the Invention

The oxidation of alkanes and other aliphatic hydrocarbons catalyzed by transition metal complexes in the liquid phase is well known in the art, and commercial applications of this technology are extensive. See, for example, J. E. Lyons, Hydrocarbon Processing, November, 1980, page 107, Table I.

However, the oxidation of unactivated hydrocarbons such as methane, ethane, propane, butanes and the like by air or $O_2$ as the oxidant is extremely difficult to achieve. The use of macrocyclic metal complexes such as metalloporphyrins as catalysts in the liquid phase has not been successful in giving rapid rates and high selectivities under mild conditions using air or $O_2$ as the oxidant. Some success has been achieved using two less economically desirable approaches:

1) The use of metalloporphyrin catalysts such as Fe(TPP)Cl and Mn(TPP)Cl (where TPP = the dianion of 5, 10, 15, 20-tetraphenylporphine) with iodosylbenzene, sodium hypochlorite, alkylhydroperoxides or other expensive, non-regenerable oxidants. [P. Traylor, D. Dolphin, and T. Traylor, J. Chem. Soc. Chem. Comm., 279 (1984); J. Groves, W. Kruper, Jr., R. Haushalter, J. Am. Chem. Soc., 102, 6377 (1980); C. Hill, B. Schardt, J. Am. Chem. Soc., 102, 6374 (1980); J. Smegal and C. Hill, J. Am. Chem. Soc., 105, 3515 (1983); A. Middleton and D. Smith, U.S. Patent 4,459,427 (July 10, 1984) EP-A-0 079 705]; or

2) The use of metalloporphyrin catalysts with molecular oxygen as oxidant and simultaneous addition of a reductant such as $NaBH_4$, ascorbic acid or colloidal platinum with $H_2$. Again, the added reagents are expensive and non-regenerable. Examples of this approach can be found in D. Mansuy, M. Fontecave and J. Bartoli, J. Chem. Soc. Chem., Comm. 253 (1983); I. Tabushi and A. Yazaki, J. Am. Chem. Soc., 103, 7371 (1981).

It is, therefore, an object of this invention to provide an azide- or nitride-activated metal coordination complex-catalyzed process for the oxidation of hydrocarbons, and particularly alkanes, using air or oxygen, but without the need for added expensive, non-regenerable oxidants, reductants, or other co-catalysts.

A further object of this invention is to provide certain novel azide-activated metal coordination complex catalysts per se for use in said process.

### SUMMARY OF THE INVENTION

The present invention provides a process for the selective oxidation of hydrocarbons or partially oxidized hydrocarbons which comprises contacting said hydrocarbons or partially oxidized hydrocarbons with air or oxygen in the presence of a catalyst comprising an azide- or nitride-activated metal coordination complex.

In accordance with the present invention, it has now been found that hydrocarbons generally, and alkanes in particular, desirably those hydrocarbons having from about 1 to 20 carbon atoms, and preferably those having from 1 to 10 carbon atoms, may readily be oxidized with air or oxygen to selectively form the corresponding hydrocarbon oxidation products such as acids, alcohols, ketones, esters, and the like, or mixtures thereof, when the catalyst is chosen from certain azide- or nitride-activated metal coordination complexes, as defined below. More particularly, it has been found that coordinating an azide or nitride ion to certain metal coordination complexes can convert a complex which is otherwise catalytically inactive, or has low catalytic activity, into a highly active catalyst for the selective oxidation of difficult-to-oxidize alkanes to form alcohols, ketones, or mixtures thereof, in good yield with little burn to carbon oxides.

By virtue of the use of these catalysts in the oxidation of hydrocarbons, and especially alkanes, many surprising and unexpected advantages accrue. For example, the reaction can be carried out at lower temperatures than heretofore employed; there is often little or no cleavage of the starting material; there is little or no burn to form CO or $CO_2$; there is higher selectivity for alcohols, when alcohols are the desired product; the reaction rates are generally faster than those of comparable prior art processes; and the processes themselves are less expensive than those of the prior art which require strong oxidants. In some instances, such as the oxidation of ethane, propane, and the like, selective oxidations can be performed

which have not been achieved to date, using the coordination complexes of this invention.

DESCRIPTION OF THE INVENTION

The process of this invention, which is applicable to hydrocarbons of virtually unlimited carbon atom content, is uniquely applicable to alkanes, which are known to be more difficult to oxidize than other types of hydrocarbons. However, it will be understood that the aforesaid catalysts are equally effective in the oxidation of other classes of hydrocarbons as well, especially those containing substituents which will enhance the reactivity of the carbon-hydrogen bond with oxygen, i.e. "activated hydrocarbons", as described below.

As aforestated, this process is particularly effective in the oxidation of alkanes, including cycloalkanes, substituted alkanes and the like. The alkane starting materials thus include straight and branch-chain compounds having from about 1 to 20 carbon atoms, preferably 1 to 10 carbon atoms such as methane, ethane, propane, n-butane, isobutane, n-pentane, n-hexane, 3-methylpentane, 2-methylpentane, heptane, 2-methylheptane, 3-methylheptane and the like, as well as cycloalkanes having from about 5 to 20 carbon atoms, preferably 5 to 10 carbon atoms, such as cyclohexane, cyclopentane, cycloheptane, cyclooctane, and the like. These compounds, if desired, may be substituted with various moieties, although care should be taken to exclude substitutents which will adversely affect the activity of the catalyst.

When the foregoing alkanes are oxidized in accordance with the process of this invention, the corresponding alcohols, ketones, and the like are obtained. Thus, this process is generally applicable to the preparation of a broad class of known materials which may be used, for example, as solvents, chemical intermediates, commodity chemicals, polymer intermediates, gasoline additives, and the like.

Illustrations of activated hydrocarbons which may also be oxidized by the process of this invention include such compounds as toluene, xylenes, cumene, ethylbenzene, diphenylmethane, fluorene, and like alkyl-substituted aromatics having from about 7 to 20 carbon atoms, preferably 7 to 12 carbon atoms. Also included are olefinic hydrocarbons, particularly those containing allylic bonds, as, for example, propylene, butenes, cyclohexene, and the like. In addition, it should be understood that the catalysts of this process are able to oxidize olefinic double bonds directly in many instances to give epoxides, ketones and alcohols, which are also useful as solvents, chemical intermediates, and the like. The olefins desirably have from about 2 to 20 carbon atoms, preferably 2 to 8 carbon atoms.

Finally, the process of this invention is also applicable to the further oxidation of partially oxidized hydrocarbons other than, of course, organic acids. Thus, for example, partially-oxidized hydrocarbons such as alcohols and aldehydes may be oxidized to a more highly oxidized state, using the catalysts of this invention. Generally these partially oxidized hydrocarbons have from about 1 to 20 carbon atoms, that is, they are the same hydrocarbons as described above except for being partially oxidized.

Thus, from the foregoing description of the starting materials, it will be seen that this process is widely applicable to a broad range of oxidizable hydrocarbons, of which the oxidation of alkanes represents a preferred embodiment of this invention. As stated above, these hydrocarbons may contain various substituents on them as long as they do not adversely affect the activity of the catalyst.

The oxidation, which may be carried out in a generally known manner, is desirably conducted in the liquid phase, using such organic solvents as benzene, acetic acid, acetonitrile, methyl acetate, or like solvents which are inert to the conditions of the reactions, or in a neat solution of the hydrocarbon if it is liquid, and pressures ranging from about 15 to 1500 psig, preferably 30 to 750 psig, at temperatures of from about 25 to 250 ° C, more preferably 70 to 180 ° C. Depending upon whether the hydrocarbon to be oxidized is a solid, liquid, or gas, it is dissolved in or bubbled through the solvent, together with air or oxygen, in the presence of the aforementioned azide-or nitride-activated metal coordination complex catalyst for periods of time sufficient to yield the desired oxidized product, generally from about 0.5 to 100 hours, and more preferably from 1 to 10 hours.

The nature of the solvent, while not critical, can have an effect on the rates and selectivities obtained and should be selected carefully in order to optimize the desired results. For example, it has been found that solvents such as acetonitrile and acetic acid are often very effective for the oxidation of alkanes to form oxygen-containing compounds, whereas reactions carried out in such solvents as methyl acetate or benzene may occur more slowly. Thus, by routine experimentation the optimum solvent for the particular process can readily be determined.

The ratios of the various reactants may vary widely, and are not critical. For example, the amount of catalyst employed can range from about $10^{-6}$ to $10^{-3}$ moles per mole of hydrocarbon such as alkane, and more preferably from about $10^{-5}$ to $10^{-4}$ moles of catalyst per mole of hydrocarbon, although other amounts are not precluded; while the amount of oxygen relative to the hydrocarbon starting material may

vary widely, generally $10^{-2}$ to $10^{2}$ moles of oxygen per mole of hydrocarbon. Care should be taken since some of the ratios fall within explosive limits. As a group, the catalysts are almost always soluble unless used in large excess. Thus, as a rule the reactions are generally carried out as solution phase reactions.

Many of the catalysts employed in this process are generally known compounds, or else may readily be prepared in accordance with established methods. These catalysts, as mentioned above, may best be defined as azide-or nitride-activated metal coordination complexes having the following general structure:

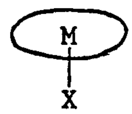

wherein M is a metal in the transition series from Groups IV(b) to VIII, such as Ti, V, Cr, Mn, Fe, Co, Nb, Mo, Ru, Rh, W, Os, Ir, or the like; X is side ($N_3^-$) or nitride ($N^{3-}$); the component depicted as "⬭" comprises a ligand such as tetraphenylporphyrin, related porphyrinate ligands, porphycenes, porphenes, phthalocyanines, 1,3-bis (2-pyridylimino) isoindoline ("BPI"), and other 1,3-bis (arylimino) isoindolines, acetylacetonates, acetates, hydroxides, or a Schiff base such as salen, saleph, or the like. Thus, by the term "ligand", as used herein, is meant any group or system of atoms coordinated to a transition metal center which forms one or more bonds to the metal, as defined above, i.e. forms a coordination complex, and stabilizes this transition metal coordination complex in desirable oxidation states. Preferred among these ligands are such macrocyclic groups as porphyrins, phthalocyanines, BPI, 1,3-bis (arylimino) isoindolines, Schiff bases, and the like. Examples of other ligands which may be employed in the catalysts of this invention are such mono-, bi-, tri-, and tetradentate ligand systems as: hydroxides, acetates, propanates, butyrates, benzoates, naphthenates, stearates, acetylacetonates, and other -diketones, 1, 3- bis (arylimino) isoindolinates, salen, saleph, porphyrinates, porphycenates, porphenates, phthalocyanates, and like systems.

In addition to the foregoing ligands there may also be employed in the catalyst of this invention such other ligands as bipyridines, terpyridines, phenanthrolines, dithiocarbamates, xanthates, salicylaldimines, cyclam, dioxocyclams, pyrazoylborates, and tetraazamacrocycles such as tetramethyltetraazadibensocycloheptadecane.

It is known in the art to halogenate ligands such as those described above in order to increase the oxidation resistance thereof, which thereby improves catalyst life. Usually, the halogen is chlorine or fluorine as in tetrachlorotetraphenylporphorinato. As used herein, the term ligand includes the halogenated type also.

The nature of the X group, namely azide or nitride, which comprises the third component of the catalysts of this invention significantly affects the activity of the final catalyst. Surprisingly, other known groups such as chloride, acetate, benzoate and the like provide very poor, if any, results and should be avoided in the oxidation of most alkanes. While applicants do not wish to be bound by any particular theories, it is believed that the reason that the azide or nitride group is effective for purposes of activating the metal complexes of this invention is due to its electron-donating properties with respect to the transition metal component.

It has also been found that a modified form of the above-described azide-and nitride-actived metal coordination complex, i.e. a dimer of said complex as defined below, is often, particularly with the nitride catalyst, likewise an effective oxidation catalyst for hydrocarbons, especially alkanes and cycloalkanes and should be regarded as the equivalent thereof. These dimers, which are closely related to the above catalysts, have the structural formula:

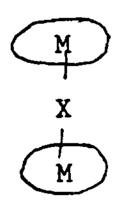

wherein M, X, and "⬭" are as defined above, i.e. M is a transition metal, X is azide or nitride, and "⬭" is a ligand. An example is the dimer of the azide of cobalt acetonylacetate.

In a further embodiment of this invention it has been found that non-ligated nitrides of certain transition metals are also surprisingly effective as catalysts in the oxidation of the aforedescribed hydrocarbons, and particularly alkanes and cycloalkanes, preferably those having from about 1 to 20 carbon atoms.

These catalysts may be defined as the transition metal nitrides of Groups IV(b) through VIII. Particularly effective among these are the nitrides of iron, manganese, chromium, and vanadium, and vanadium is the most preferred. The activity of these compounds as oxidation catalysts using air or oxygen under mild operating conditions in the liquid phase is quite surprising because they are virtually insoluble in the liquid medium, and thought to be quite stable.

The oxidation of hydrocarbons, most preferably alkanes, using these catalysts may be carried out in the same general way as the aforedescribed process employing the nitride metal complexes. That is to say, these catalysts are effective at temperatures of from about 75 to 250 °C, preferably 100 to 200 °C, with pressures of from about 15 to 1500 psig, preferably 30 to 750 psig. Again, depending upon whether the hydrocarbon is a solid, liquid, or gas, it is dissolved in or bubbled through a solvent such as benzene or acetic acid, or through the liquid hydrocarbon, neat, together with air or oxygen, for periods of time necessary to provide the desired product. The ratio of catalyst to substrate may vary, but is generally from about $10^{-6}$ to $10^{-1}$ moles of catalyst per mole of hydrocarbon.

The products may range from alcohols and ketones to aldehydes and acids, but in the case of alkanes, they are generally alcohols and ketones, with little burn to CO and $CO_2$.

The catalysts described and employed herein are either known or can readily be prepared by procedures described in the art, starting with known metal coordination complexes and/or literature preparations for making such complexes. In most cases the preparation of the transition metal azide complexes of this invention involves reactions between known complexes having a metal halide, acetate, hydroxide or similar groups with either hydrazoic acid or sodium azide.

For example, Mn(TPP)Cl, Fe(TPP)Cl, and Cr(TPP)Cl metal complexes may be prepared by a standard method in which $TPPH_2$ (in which "TPP" is tetraphenylporphyrinato) and $Mn^{2+}$, $Fe^{2+}$, or $Cr^{3+}$ salts are refluxed together in a dimethylformamide solution. Purification is achieved by chromotography. (See, e.g., A. D. Adler et al, J. Inorg. Nucl. Chem., 32, 2443 (1970).) From these metal salts the corresponding azides may be prepared by metathesis reactions with dissolved $NaN_3$ or hydrazoic acid.

Similarly, $Mn(TPP)N_3$, $Cr(TPP)N_3$, $Mn(Por)N_3$, and $Cr(Por)N_3$ (where "Por" is porphyrinato) can be synthesized by the reaction of hydrazoic acid with the corresponding hydroxide of the metal coordination complex. (See, e.g., J. W. Buchler et al, Z. Naturforsch, 39b, 222-230 (1984) for preparation of these metal complexes.)

In a like manner, $Fe(TPP)N_3$ may be synthesized by reacting Fe(TPP)-O-(TPP)Fe with hydrazoic acid. (See D. A. Summerville et al, JACS, 98, 1747-1752 (1976).)

Also, Co(BPI)OAc (where "BPI" is 1,3-bis (2-pyridylimino) isoindoline) may be synthesized by the condensation of 1,2-dicyanobenzene with 2-aminopyridine in the presence of cobaltous acetate. Other 1, 3-bis (arylimino) isoindolines are prepared similarly. (See W. O. Siegl, J. Org. Chem., 42, 1872-78 (1977).) $Co(BPI)N_3$ may then be formed by reacting Co(BPI)OAc with $NaN_3$ in solution.

It is also possible to prepare azide transition metal coordination complexes by direct addition of azide ions. For example, novel metal phthalocyanine ("Pc") azide complexes such as $Cr(Pc)N_3$ and $Mn(Pc)N_3$ may be prepared by the following technique: In a Soxhlet extractor 400 ml of $NaN_3$ saturated anhydrous ethanol solution is refluxed under $N_2$. In the thimble is placed 2.0g of Cr(Pc) or Mn(Pc). Reflux is continued until all of the solid Cr(Pc) or Mn (Pc) is extracted into the alcohol. The solution is filtered, evaporated to dryness and the solid $Cr(Pc)N_3$ or $Mn(Pc)N_3$ is washed thoroughly with distilled water to remove any residual $NaN_3$. Structures are confirmed by visible and infrared spectra:

| Complex | N-N Stretch in IR |
|---|---|
| $Cr(Pc)N_3$ | 2052 $cm^{-1}$ |
| $Mn(Pc)N_3$ | 2047 $cm^{-1}$ |

In a like manner those skilled in the art can readily prepare other azide catalysts by the general procedures and literature teachings described above.

In most cases, the preparation of the metal nitride catalysts of this invention involves either photolysis or thermolysis of the corresponding transition metal azide complexes, or high temperature reactions of metal ligand complexes with sodium azide which forms the nitride in one step.

Illustrations of the preparation of certain of the nitride-activated metal coordination complexes of this invention are as follows:

Mn(TPP)N or Cr(TPP)N can be synthesized by the photodissociation of their corresponding azide complexes in benzene or THF solvent. (See J. W. Buchler et al, Z. Naturforsch., 39b 222-230 (1984).)

Mn(TPP)N and Cr(TPP)N can also be prepared by the action of ammonia and hypochlorite ion on Mn-(TPP)OH and Cr(TPP)OH respectively. (See J.W. Buchler et al, Inorg. Chem., 22, 879-884 (1985). Nearly all nitride complexes are prepared by either the decomposition of azido complexes or the reduction of ammonia complexes.

Alternatively, the dimer $[Fe(TPP)]_2N$, for example, can be prepared by the thermal decomposition of Fe-$(TPP)N_3$ in xylene. (See D. A. Summerville et al, JACS, 98, 1747-52 (1976).) Also, the dimer $[Fe(Pc)]_2N$ can be prepared by the reaction of Fe(Pc) with $NaN_3$ in refluxing chloronaphthalene. (See L. A. Bottomley et al, Inorg. Chem., 24, 3733-37 (1985).)

In a like manner those skilled in the art can readily prepare other nitride catalysts by the general procedures and literature teachings described above, employing the corresponding ligated azides described above as the starting material.

While the effectiveness of a particular catalyst may depend in part on the nature of the hydrocarbon starting material, selection of the catalyst for oxidising any particular hydrocarbon can be readily determined by those skilled in the art. Examples of those catalysts which are most preferred, particularly for oxidation of lower alkanes, include such compounds as tetraphenylporphyrinato manganese (III) azide, tetraphenylporphyrinato manganese (V) nitride, tetraphenylporphyrinato chromium (III) azide, tetraphenylporphyrinato chromium (V) nitride, phthalocyaninato manganese (III) azide, phthalocyaninato chromium (III) aside, ($\mu$-nitrido)bis(phthalocyaninato) iron (III$\frac{1}{2}$), and the like.

The process of this invention will now be illustrated by, but is not intended to be limited to, the following examples.

## EXAMPLES

A series of runs were carried out employing a variety of catalysts, alkanes, solvents, and operating conditions. Examples 1-69 show the comparative efficacy of azide coordination catalysts, and example 70-88 show the comparative advantages of nitride coordination catalysts. Examples 89-121 demonstrate the unexpected activity of unligated nitrides.

Except where shown otherwise in the tables, these runs were carried out as follows: the alkane was dissolved in an appropriate solvent containing the catalyst, and oxygen was added to the desired pressure. Oxidation was carried out at the designated temperature for the time listed in the tables. Gases and liquid products were analyzed by gas chromatography (GC) and mass spectroscopy (MS).

In the following examples, TBA and IPA are t-butyl and isopropyl alcohol respectively, (acac) is acetylacetonate, TPP is tetraphenylporphorinato, Pc is phthalocyaninato. Activity is measured in terms of "turn over number" (T.O.N.) which is defined, unless otherwise indicated, as moles of $O_2$ consumed/mole of catalyst in Tables I-IV and as moles of product/mole of catalyst in Tables V-IX. Selectivity is defined as moles TBA product x 100/moles of isobutane reacted.

TABLE I

EFFECTS OF AZIDE ON COBALT - CATALYZED OXIDATION OF ISOBUTANE[a)]

| EXAMPLE | CATALYST - mmoles | | $O_2$ UPTAKE (mmoles) | T.O.N. | PRODUCTS (mmoles) ACETONE | PRODUCTS (mmoles) TBA | $O_2$ COVERTED TO ACETONE + TBA (mmoles) | ISOBUTANE REACTED - (MOL%) | SELECTIVITY |
|---|---|---|---|---|---|---|---|---|---|
| 1 | None | - | 0 | - | 0 | 0 | 0 | 0 | - |
| 2 | AIBN[b)] | 1.0 | 4.9 | 5 | 1.2 | 4.2 | 5.1 | 3.5 | 78 |
| 3 | AIBN | 0.10 | 1.7 | 17 | 0.3 | 0.7 | 1.1 | 1.0 | 70 |
| 4 | $Co(acac)_3$ | 0.10 | 1.5 | 15 | 0.3 | 2.1 | 1.8 | 2.2 | 88 |
| 5 | $Co(acac)_3$ | 0.025 | 0.9 | 36 | 0.2 | 0.8 | 0.9 | 0.9 | 80 |
| 6 | $Co(acac)_3$ | 0.05 | 3.7 | 74 | 0.3 | 2.2 | 1.9 | 2.2 | 88 |
| | $+NaN_3$ | 0.05 | | | | | | | |
| 7 | $Co(acac)_2$ | 0.10 | 5.2 | 52 | 0.54 | 4.5 | 3.1 | 4.0 | 89 |
| 8 | $Co(acac)_2$ | 0.05 | 4.6 | 93 | 0.24 | 1.9 | 1.3 | 1.9 | 89 |
| | $+ NaN_3$ | 0.05 | | | | | | | |
| 9 | Co(BPI)OAc | 0.025 | 0.6 | 24 | Low | Low | Low | Low | NA |
| 10 | $Co(BPI)N_3$ | 0.025 | 4.9 | 196 | 0.7 | 4.7 | 4.1 | 5.2 | 87 |

a) Isobutane, 6-7 grams, in 25 ml benzene, oxidized at 80°C, $O_2$ partial pressure - about 75 psig, for 6 hours.

b) AIBN = azobisisobutyronitrile

TABLE II

EFFECTS OF AZIDE ON MANGANESE-CATALYZED OXIDATION OF ISOBUTANE[a)]

| EXAMPLE | CATALYST | - mmoles | $O_2$ UPTAKE (mmoles) | T.O.N. | PRODUCTS (mmoles) ACETONE | TBA | $O_2$ COVERTED TO ACETONE + TBA (mmoles) | ISOBUTANE REACTED - (MOL%) | SELECTIVITY |
|---|---|---|---|---|---|---|---|---|---|
| 11 | $Mn(acac)_3$ | 0.025 | 0 | - | 0 | 0 | 0 | 0 | - |
| 12 | Mn(TPP)Cl | 0.05 | 0 | - | 0 | 0 | 0 | 0 | - |
| 13 | Mn(TPP)OAc | 0.05 | 0 | - | 0 | 0 | 0 | 0 | - |
| 14 | $Mn(TPP)N_3$ | 0.025 | 5.5 | 220 | 0.7 | 6.2 | 4.9 | 5.6 | 90 |
| 15 | $Mn(TPP)N_3$ | 0.013 | 2.3 | 177 | 0.4 | 3.0 | 2.5 | 2.7 | 88 |
| 16 | $Mn(Pc)N_3$ | 0.028 | 4.5 | 161 | 0.6 | 4.2 | 3.6 | 4.5 | 88 |
| 17 | $Mn(Pc)N_3$ | 0.013 | 3.7 | 285 | NA | NA | NA | NA | NA |
| 18 | $Mn(Pc)N_3$ | 0.007 | 2.3 | 330 | NA | NA | NA | NA | NA |

a) Isobutane, 6-7 grams, in 25 ml benzene, oxidized at 80°C, $O_2$ partial pressure - about 75 psig, for 6 hours.

TABLE III

EFFECTS OF AZIDE ON CHROMIUM-CATALYZED OXIDATION OF ISOBUTANE[a)]

| EXAMPLE | CATALYST - mmoles | | $O_2$ UPTAKE (mmoles) | T.O.N. | PRODUCTS (mmoles) ACETONE | TBA | $O_2$ COVERTED TO ACETONE + TBA (mmoles) | ISOBUTANE REACTED - (MOL%) | SELECTIVITY |
|---|---|---|---|---|---|---|---|---|---|
| 19 | $Cr(acac)_3$ | 0.025 | 0 | - | 0 | 0 | 0 | 0 | - |
| 20 | Cr(TPP)Cl | 0.025 | 0 | - | 0 | 0 | 0 | 0 | - |
| 21 | $Cr(TPP)N_3$ | 0.013 | 3.4 | 262 | 0.4 | 3.3 | 2.7 | 3.5 | 89 |
| 22 | $Cr(TPP)N_3$ | 0.025 | 6.6 | 264 | 0.8 | 6.3 | 5.2 | 6.7 | 88 |
| 23 | $Cr(TPP)N_3$ | 0.040 | 8.7 | 218 | 1.3 | 8.7 | 7.5 | 7.7 | 87 |
| 24 | $Cr(acac)_3$ | 0.05 | 2.6 | 52 | NA | NA | NA | NA | NA |

a) Isobutane, 6-7 grams, in 25 ml benzene, oxidized at 80°C, $O_2$ partial pressure - about 75 psig, for 6 hours.

TABLE IV

OXIDATION OF ISOBUTANE AT 100°C USING FIRST ROW METAL PORPHYRINATO AZIDES AS CATALYSTS[a)]

| EXAMPLE | CATALYST - mmoles | | SOLVENT | $i-C_4H_{10}$ (mmoles) | REACT. TIME (hrs.) | $O_2$ UPTAKE (mmoles) | T.O.N. | PRODUCTS (mmoles) | | ISOBUTANE CONVERTED TO (ACETONE + TBA) (Mol%) |
|---|---|---|---|---|---|---|---|---|---|---|
| | | | | | | | | ACETONE | TBA | |
| 25 | None | - | Benzene | 117 | 6 | 0 | - | 0 | 0 | - |
| 26 | $Cr(TPP)N_3$ | 0.042 | Benzene | 105 | 2.5 | 7.2 | 172 | 1.3 | 3.7 | 4.8 |
| 27 | $Mn(TPP)N_3$ | 0.043 | Benzene | 103 | 6 | 11.9 | 282 | 1.9 | 7.9 | 9.5 |
| 28 | $Fe(TPP)N_3$ | 0.042 | Benzene | 116 | 6 | 3.8 | 90 | 1.5 | 3.1 | 4.0 |
| 29 | $Cr(TPP)N_3$ | 0.042 | 1/1 Benzene/$CH_3CN$ | 81 | 5 | 5.2 | 124 | 1.6 | 2.8 | 5.4 |
| 30 | $Mn(TPP)N_3$ | 0.042 | 1/1 Benzene/$CH_3CN$ | 66 | 7 | 10.1 | 241 | 2.6 | 5.5 | 12.3 |

a) Isobutane was dissolved in 25 ml of solvent containing the catalyst. Oxygen was added and oxidations were run at 100°C and 110-90 psig. Gas and liquid product analysis were done by GC.

TABLE V

EFFECTS OF AZIDE ON METAL CATALYZED OXIDATION OF PROPANE[a)]

| EXAMPLE | CATALYST - moles/1 | | REACTION TIME(HRS) | PRODUCTS mmole/gram Reaction Mixture ACETONE | IPA | T.O.N Mole Product/ Mole Catalyst |
|---|---|---|---|---|---|---|
| 31 | Co(acac)$_3$ | 0.032 | 5.0 | 0.14 | 0.19 | 57 |
| 32 | Co(acac)$_3$ | 0.032 | 12.8 | 0.26 | 0.15 | 89 |
| 33 | Co(acac)$_3$ | 0.032 | 60.5 | 0.23 | 0.17 | 87 |
| 34 | Co(BPI)N$_3$ | 0.02 | 13.1 | 0.24 | 0.13 | 132 |
| 35 | Co(BPI)N$_3$ | 0.02 | 61.5 | 0.24 | 0.13 | 131 |
| 36 | Mn(Pc)N$_3$ | 0.006 | 14.1 | 0.18 | 0.13 | 362 |
| 37 | Cr(TPP)N$_3$ | 0.006 | 13.4 | 0.20 | 0.12 | 377 |

a) Catalyst dissolved in 7 ml benzene. Propane oxidized by air at 80 bars total pressure, i.e. about 1200 psig, at 150°C.

From the foregoing results in Tables I-V it will be seen that (1) reactions run with no catalyst give no products; (2) initiators and coordination complexes alone give, at best, modest activity as shown by the turn over number; (3) by adding an aside such as sodium azide separately improves activity (T.O.N.); while coordinating aside with the metal complex gives the best results, e.g. Examples 10 and 14-18.

These results are clearly demonstrated, for example, in Table II where a typical manganese complex, $Mn(acac)_2$, or Mn (TPP) halides, acetates and the like (e.g. Exs. 11-13), give no oxidation. However, replacing the Cl or OAc with $N_3$ provides a startlingly active isobutane oxidation catalyst. This pattern is clearly repeated in Tables III to V as well as in the following examples.

EXAMPLES 38-40

Hexane, 15 ml, was oxidized in acetic acid, 15 ml, using 42 mg. of cobalt catalyst (see Exs. 38-40). The oxygen pressure was 100 psig and the reaction temperature was 100 ° C. The major reaction products as determined by standardized GC were 2-and 3-hexanol, 2- and 3-hexanone together with some $C_2$-$C_4$ carboxylic acids produced by C-C bond cleavage reactions. The catalytic activity, as measured by the moles $O_2$ taken up per mole of catalyst over the 6 hour reaction time is given in the examples below. It can be seen that while Co(BPI)OAc was inactive, adding sodium azide produced an active catalyst, and replacing acetate by coordinated aside, i.e. $Co(BPI)N_3$, produced the highest activity.

| Example | Catalyst | (T.O.N.) Moles $O_2$ Uptake/Mole Cat. |
|---|---|---|
| 38 | Co(BPI)(OAc) | 0.0 |
| 39 | Co(BPI)(OAc) + $NaN_3$ (13 mg) | 20.2 |
| 40 | $Co(BPI)N_3$ | 26.0 |

EXAMPLES 41-61

Under the conditions listed in Table VI below, cyclohexane was oxidized to a mixture of cyclohexanol and cyclohexanone. Although conventional metal TPP complexes such as the chlorides, acetates and the like were completely inactive (with the exception of Fe(TPP)OAc which has some activity), $Cr(TPP)N_3$, Fe-$(TPP)N_3$ and $Mn(TPP)N_3$ were quite active catalysts.

TABLE VI
CYCLOHEXANE OXIDATION[a)]
USING METALLOPORPHYRIN CATALYSTS

| EXAMPLE | CATALYST - mmole | | SOLVENT | PRESSURE (psig) | PRODUCT (mmole) CYCLOHEXANOL | CYCLOHEXANONE | T.O.N.[b)] | AVE. T.O.N./HR |
|---|---|---|---|---|---|---|---|---|
| 41 | Cr(TPP)Cl | 0.175 | neat | 500 | 0.00 | 0.00 | 0 | 0 |
| 42 | $Cr(TPP)N_3$ | 0.088 | neat | 500 | 5.90 | 5.32 | 128 | 28 |
| 43 | $Cr(TPP)N_3$ | 0.088 | neat | 500 | 5.25 | 5.52 | 122 | 28 |
| 44 | $Cr(TPP)N_3$ | 0.088 | neat | 125 | 6.82 | 9.22 | 182 | 36 |
| 45 | $Cr(TPP)N_3$ | 0.088 | neat | 250 | 5.34 | 7.74 | 149 | 27 |
| 46 | $Cr(TPP)N_3$ | 0.173 | neat | 500 | 6.95 | 8.67 | 106 | 12 |
| 47 | $Cr(TPP)N_3$ | 0.044 | neat | 500 | 3.01 | 3.55 | 149 | 25 |
| 48 | $Cr(TPP)N_3$ | 0.088 | neat | 500 | 5.34 | 5.78 | 126 | 27 |
| 49 | Fe(TPP)Cl | 0.175 | neat | 500 | 0.00 | 0.00 | 0 | 0 |
| 50 | $Fe(TPP)N_3$ | 0.088 | neat | 500 | 3.01 | 3.97 | 79 | 18 |
| 51 | $Fe(TPP)N_3$ | 0.088 | neat | 500 | 8.19 | 11.00 | 218 | 18 |
| 52 | $Fe(TPP)N_3$ | 0.088 | neat | 500 | 7.30 | 9.62 | 192 | 48 |
| 53 | $Fe(TPP)N_3$ | 0.173 | neat | 500 | 7.95 | 10.15 | 105 | 16 |
| 54 | Fe(TPP)OAc | 0.173 | neat | 500 | 12.90 | 9.55 | 130 | 12 |
| 55 | Mn(TPP)OAc | 0.250 | neat | 500 | 0.00 | 0.00 | 0 | 0 |
| 56 | Mn(Pc) | 0.250 | neat | 500 | 0.00 | 0.00 | 0 | 0 |
| 57 | $Mn(TPP)N_3$ | 0.173 | neat | 500 | 2.79 | 2.85 | 32 | 6 |
| 58 | $Mn(TPP)N_3$ | 0.175 | $CH_3CN$ | 500 | 2.40 | 2.60 | 29 | 4 |
| 59 | $Mn(TPP)N_3$ | 0.088 | neat | 500 | 2.67 | 3.42 | 69 | 10 |
| 60 | $Mn(TPP)N_3$ | 0.173 | neat | 500 | 12.30 | 20.80 | 191 | 10 |
| 61 | $Mn(acac)_3$ | 0.175 | neat | 500 | 0.25 | 1.50 | 20 | 2 |

a) CONDITIONS: 100cc solvent; 10% oxygen in nitrogen at a flow rate of 5-10cc/min. Temp. = 100°C except Ex. 52, which is 125°C.

b) T.O.N. = moles of product (cyclohexanol and cyclohexanone)/mole of catalyst.

## EXAMPLE 62

A 30 ml Fisher-Porter Aerosol tube equipped with thermocouple, magnetic stirrer and gas inlet and sampling ports was charged with 17 mg of chromiumtetraphenylporphyrinatoazide and 30 ml of 2,3-dimethylbutene-2. After thorough flushing with oxygen, 60 psig of oxygen pressure was admitted to the aerosol tube and the tube heated to 80 ° C with stirring. Reaction was run at 80 ° C with vigorous stirring for three hours during which time the pressure was kept at 100 psig by continous addition of 10 psi increments of oxygen as it was consumed. After 3 hours, 210 mmoles of oxygen was consumed. GC analysis of the reaction product showed that about 200 mmoles of 2, 3-dimethylbutene-2 had been consumed to produce four major products: acetone, tetramethylethylene oxide (TMEO), 2,3-dimethyl-2-hydroxybutenel-1 and 2, 3-dimethyl-2-hydroperoxybutene-1 with tetramethylethylene oxide being the predominant product.

EXAMPLE 63

Under the conditions of Example 62, but substituting chromiumphthalocyaninatoazide as catalyst, there were obtained fair yields of acetone, TMEO, 2,3-dimethyl-2-hydroxybutene-1 and 2,3-dimethyl-2-hydroperoxybutene-1.

EXAMPLE 64

Under the conditions of Example 62, but substituting manganesephthalocyaninatoazide as catalyst, high yields of acetone, TMEO, 2,3-dimethyl-2-hydroxybutene-1 and 2,3-dimethyl-2-hydroperoxybutene-1 were obtained.

EXAMPLE 65

Under the conditions of Example 62 but substituting manganesetetraphenylporphyrinatoazide as catalyst, high yields of acetone, TMEO, 2,3-dimethyl-2-hydroxybutene-1 and 2,3-dimethyl-2-hydroperoxybutene-1 were obtained.

EXAMPLE 66

Under the conditions of Example 65 except that reaction temperature was 22 ° C, 11% conversion of 2,3-dimethylbutene-2 was obtained and the major reaction products were acetone, TMEO, 2,3-dimethyl-2-hydroxybutene-1 and 2, 3-dimethyl-2-hydroperoxybutene-1.

EXAMPLE 67

Under the conditions of Example 64 except that reaction temperature was 22 ° C, 2,3-dimethylbutene-2 was converted to acetone, TMEO, 2, 3-dimethyl-2-hydroxybutene-1 and 2,3-dimethyl-2-hydroperoxybutene-1.

EXAMPLE 68

Under the conditions of Example 62 except that reaction temperature was 22 ° C, 2,3-dimethylbutene-2 was converted to acetone, TMEO, 2, 3-dimethyl-2-hydroxybutene-1 and 2,3-dimethyl-2-hydroperoxybutene-1.

EXAMPLE 69

Under the conditions of Example 35 using $Mn(Pc)N_3$ as catalyst, ethane was oxidized to a small extent.

EXAMPLES 70-74

Propane was oxidized in a small, glass-lined rocking autoclave under the conditions indicated in Table VII. Tetraphenylporphorinatomanganese (V) nitride was compared with a typical autoxidation catalyst: Co-$(acac)_3$.

TABLE VII

| EFFECTS OF NITRIDE ON METAL CATALYZED OXIDATION OF PROPANE[a] | | | | | | |
|---|---|---|---|---|---|---|
| EXAMPLE | CATALYST - | moles/l | REACTION TIME/HRS. | PRODUCT[b] | | |
| | | | | ACETONE | IPA | T.O.N. |
| 70 | $Co(acac)_3$ | 0.032 | 5.0 | 0.14 | 0.19 | 57 |
| 71 | $Co(acac)_3$ | 0.032 | 12.8 | 0.26 | 0.15 | 89 |
| 72 | $Co(acac)_3$ | 0.032 | 60.5 | 0.23 | 0.17 | 87 |
| 73 | Mn(TPP)N | 0.006 | 13.0 | 0.18 | 0.18 | 383 |
| 74 | Mn(TPP)N | 0.006 | 61.8 | 0.41 | 0.30 | 828 |

[a] Catalyst was dissolved in 7 ml benzene. Propane was oxidized by air at about 1200 psig total pressure at 150 °C.
[b] mmoles/gram reaction mixture

EXAMPLES 75-81

Propane was oxidized in a 300 ml glass-lined stirred autoclave as indicated in Table VIII. Tetraphenylporphyrinatomanganese (V) nitride, tetraphenylporphyrinato chromium (V) nitride, and bis-phthalocyaninatoiron(III½) nitride were compared with a typical autoxidation catalyst, i.e. $Co(acac)_3$.

TABLE VIII

PROPANE OXIDATION IN 300 Ml. STIRRED AUTOCLAVE[a)]

| EXAMPLE | CATALYST | CATALYST (mM.) | BENZENE (g.) | PROPANE (mM.) | TIME (HOURS) | PRODUCTS[b)] ACETONE | IPA | T.O.N. | T.O.N. /HOUR |
|---|---|---|---|---|---|---|---|---|---|
| 75 | $Co(acac)_3$ | 0.320 | 70.32 | 568 | 2.0 | 0.190 | 0.009 | 6.3 | 3.1 |
| 76 | $Co(acac)_3$ | 0.320 | 70.32 | 568 | 8.1 | 0.024 | 0.0 | 5.3 | 0.7 |
| 77 | Mn(TPP)N | 0.060 | 70.32 | 568 | 8.0 | 0.118 | 0.010 | 156.7 | 19.6 |
| 78 | $[Fe(Pc)]_2N$ | 0.036 | 42.20 | 682 | 7.2 | 0.214 | 0.029 | 282.9 | 39.1 |
| 79 | Cr(TPP)N | 0.036 | 42.20 | 682 | 4.0 | 0.049 | 1.771 | 79.2 | 19.8 |
| 80 | Cr(TPP)N | 0.036 | 42.20 | 682 | 8.0 | 0.079 | 1.937 | 115.3 | 14.4 |
| 81 | Cr(TPP)N (200°C.) | 0.036 | 42.20 | 682 | 8.0 | 0.28 | 0.097 | 457.6 | 57.2 |

a) Reactions were performed at 150°C., unless noted. The propane was oxidized by air at about 1000 psig. The solvent was benzene.

b) mmoles/gram reaction mixture

EXAMPLES 82-88

Cyclohexane was oxidized in a 300 ml autoclave with an oxygen-containing gas constantly sparging through the system under the conditions listed on Table IX. Chromium, manganese and iron TPP

(tetraphenylporphyrinato) nitrides were catalytically active whereas the corresponding halides were not. Cyclohexanol and cyclohexanone were the organic reaction products.

TABLE IX

| CYCLOHEXANE OXIDATION[a] | | | | | | |
|---|---|---|---|---|---|---|
| EXAMPLE | CATALYST | SOLVENT | PRODUCT (mmoles) | | T.O.N | T.O.N./hr. |
| | | | CYCLOHEXANOL | CYCLOHEXANONE | | |
| 82 | Cr(TPP)Cl | neat | 0 | 0 | 0 | 0 |
| 83 | Cr(TPP)N | 50% benzene | 1.63 | 1.79 | 71 | 33 |
| 84 | Mn(TPP)Cl | neat | 0 | 0 | 0 | 0 |
| 85 | Mn(TPP)N | 50% benzene | 3.24 | 2.82 | 192 | 23 |
| 86 | Fe(TPP)Cl | neat | 0 | 0 | 0 | 0 |
| 87 | $[Fe(TPP)]_2N$ | 50% benzene | 1.97 | 3.73 | 207 | 27 |
| 88 | Co (TPP)Cl | neat | 0 | 0 | 0 | 0 |

[a] Temp. = 100 °C; pressure is 500 psig of 10% $O_2$ in $N_2$; flowing at 5-10 cc/min; catalyst = 0.01-0.09 mmole/100cc solvent.

From the foregoing results of Tables VII-IX it will readily be seen that when, for example, the acetylacetonate of a transition metal, as in Table VII and VIII, or even the chloride of a metal complex, as in Table IX, is substituted by a nitride of a metal complex, dramatic improvements in activity and yields are obtained.

EXAMPLES 89-121

In the following examples, a series of runs were carried out using the above-described transition metal nitrides on a variety of hydrocarbons under conditions described in Table X, below. Products were analyzed by GC.

These runs were carried out as follows: The catalyst and 30 ml hexane were added to a 30 ml Fisher-Porter aerosol tube equipped with a magnetic stirrer and gas inlet tubes. The reaction was carried out at 100-200 °C under 100 psig of $O_2$. The major products were 1- and 2- hexanol, and 1- and 2- hexanone with some $C_2$-$C_6$ carboxylic acids also being formed.

TABLE X

OXIDATION OF ALKANES AND CYCLOALXANES WITH METAL NITRIDES

| EXAMPLE | CATALYST | SUBSTRATE | SOLVENT | TEMP (C°) | TIME (HRS) | $O_2$ UPTAKE (MMOLES) |
|---|---|---|---|---|---|---|
| 89 | $Mn_3N$ | n-Hexane | - | 100 | 6 | 0 |
| 90 | $Mn_3N$ | n-Hexane | - | 100 | 12 | 3.6 |
| 91 | CrN | n-Hexane | - | 100 | 6 | 0 |
| 92 | CrN | n-Hexane | - | 100 | 12 | 4.2 |
| 93 | $Fe_3N$ | n-Hexane | - | 100 | 6 | 0.6 |
| 94 | $Fe_3N$ | n-Hexane | - | 120 | 6 | 1.8 |
| 95 | VN | n-Hexane | - | 100 | 6 | 3.6 |
| 96 | VN | n-Hexane | - | 120 | 6 | 22.2 |
| 97 | VN | n-Hexane | - | 120 | 6 | 19.2 |
| 98 | VN | i-Butane | Benzene | 80 | 6 | 1.5 |
| 99 | VN | i-Butane | - | 100 | 6 | 6.3 |
| 100 | VN | Cyclohexane | - | 100 | 6 | 0.3 |
| 101 | $V_2O_3$ | n-Hexane | - | 100 | 6 | 0 |
| 102 | $V_2O_3$ | n-Hexane | - | 120 | 6 | 0 |
| 103 | $V_2O_4$ | n-Hexane | - | 100 | 6 | 1.5 |
| 104 | $V_2O_4$ | n-Hexane | - | 120 | 6 | 0 |
| 105 | $V_2O_5$ | n-Hexane | - | 100 | 6 | 0 |
| 106 | $V_2O_5$ | N-Hexane | - | 120 | 6 | 1.2 |
| 107 | $CpV(CO)_4$[a)] | n-Hexane | - | 100 | 6 | 0 |
| 108 | $V(acac)_3$ | n-Hexane | - | 100 | 6 | 0 |
| 109 | $VO(acac)_2$ | n-Hexane | - | 100 | 6 | 20.4 |
| 110 | $VO(acac)_2$ | n-Hexane | - | 120 | 6 | 21.0 |
| 111 | VO(Oxalate) | n-Hexane | - | 100 | 6 | 1.8 |
| 112 | VO(Oxalate) | n-Hexane | - | 120 | 6 | 15.6 |
| 113 | VO(Pc) | n-Hexane | - | 100 | 6 | 0 |
| 114 | VN | n-Hexane | - | 120 | 3.5 | 2.4 |
| 115 | VN | n-Hexane | - | 120 | 9.5 | 14.0 |
| 116 | VN | n-Hexane | - | 120 | 3.5 | 1.8 |
| 117 | VN | n-Hexane | - | 120 | 9.5 | 3.6 |
| 118 | VN | n-Hexane | - | 120 | 3.5 | 4.8 |
| 119 | VN | n-Hexane | - | 120 | 9.5 | 9.7 |
| 120 | VN | n-Hexane | - | 120 | 3.5 | 3.0 |
| 121 | VN | n-Hexane | - | 120 | 9.5 | 14.1 |

[a)] $CpV(CO)_4$ = cyclopentadienyl $V(CO)_4$

In this table X, activity of the catalyst is measured in terms of $O_2$ uptake. From these results it can be seen that nitrides are effective oxidation catalysts, and vanadium nitride is particularly effective.

## Claims

**1.** A process for the selective oxidation of hydrocarbons or partially oxidized hydrocarbons which comprises contacting said hydrocarbons or partially oxidized hydrocarbons with air or oxygen in the presence of a catalyst comprising an azide- or nitride-activated metal coordination complex.

**2.** A process according to Claim 1, wherein the azide- or nitride-activated metal coordination complex catalyst has the formula

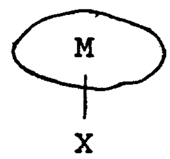

wherein M is a transition metal; X is azide or nitride; and the component "⬭" is a ligand.

**3.** A process according to Claim 1, wherein the coordination complex catalyst has the formula

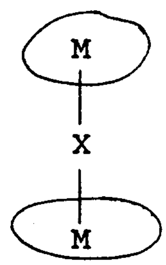

wherein M is a transition metal; X is azide or nitride, and the component "⬭" is a ligand.

**4.** A process according to Claims 2 or 3, wherein the transition metal is selected from Ti, V, Cr, Mn, Fe, Co, Nb, Mo, Ru, Rh, W, Os and Ir.

**5.** A process according to any of Claims 2 to 4, wherein the ligand is selected from tetraphenylporphyrins, porphycenes, porphenes, porphyrins, phthalocyanines, 1,3-bis(arylimino) isoindolines, acetylacetonates, acetates, hydroxides, Schiff bases, propanoates, butyrates, benzoates, naphthenates, stearates, porphyrinates, porphenates, and phthalocyanates.

**6.** A process according to any of Claims 2 to 4, wherein the ligand is selected from bipyridines, terpyridines, phenanthrolines, dithiocarbamates, xanthates, salicylaldimines, cyclam, dioxocyclams, pyrazoylborates, and tetraazamacrocycles.

**7.** A process for the oxidation of hydrocarbons or partially oxidized hydrocarbons which comprises contacting said hydrocarbons or partially oxidized hydrocarbons with air or oxygen in the presence of a catalyst comprising a transition metal nitride.

**8.** A process according to Claim 7, wherein the transition metal nitrides are selected from Groups IV through VIII nitrides.

**9.** A process according to Claim 7, wherein the nitrides are selected from manganese nitride, iron nitride, chromium nitride and vanadium nitride.

**10.** A process according to any of Claims 1 to 9, wherein the hydrocarbons have from 1 to about 20 carbon atoms.

**11.** A process according to any of Claims 1 to 10, wherein the hydrocarbons are alkanes.

**12.** A process according to Claim 11, wherein said alkanes have from 1 to about 10 carbon atoms.

**13.** A process according to any of Claims 1 to 9, wherein the hydrocarbons or partially oxidized hydrocarbons are selected from cycloalkanes, alkyl-substituted aromatics, olefins and partially oxidized derivatives thereof.

**14.** A process according to any of Claims 1 to 12, wherein the products of the oxidation are alcohols, ketones, or mixtures thereof.

**15.** A process according to any of Claims 1 to 13, wherein the products of the oxidation are alcohols, ketones, acids, esters, or mixtures thereof.

**16.** A process according to any of Claims 1 to 15, wherein the reaction is carried out in the presence of an organic solvent.

**17.** A compound selected from $Mn(Pc)N_3$ and $Cr(Pc)N_3$.

**Patentansprüche**

**1.** Verfahren zur selektiven Oxidation von Kohlenwasserstoffen oder partiell oxydierten Kohlenwasserstoffen, das darin besteht, daß die Kohlenwasserstoffe oder partiell oxydierten Kohlenwasserstoffe in Gegenwart eines Katalysators, der einen Azid- oder Nitrid-aktivierten Metallkoordinationskomplex umfaßt, mit Luft oder Sauerstoff in Kontakt gebracht werden.

**2.** Verfahren nach Anspruch 1, bei dem der Azid- oder Nitrid-aktivierte Metallkoordinationskomplex-Katalysator die Formel

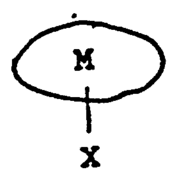

aufweist, in der M ein Übergangsmetall, X ein Azid oder Nitrid und der Bestandteil "⬭" einen Liganden bedeuten.

**3.** Verfahren nach Anspruch 1, wobei der Koordinationskomplex-Katalysator die Formel

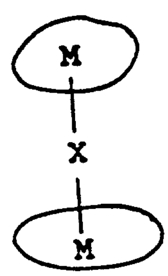

aufweist, worin M ein Übergangsmetall ist, X ein Azid oder Nitrid ist und die Komponente "⬭" einen Liganden darstellt.

**4.** Verfahren nach Anspruch 2 oder 3, wobei das Übergangsmetall unter Ti, V, Cr, Mn, Fe, Co, Nb, Mo, Ru, Rh, W, Os und Ir ausgewählt ist.

**5.** Verfahren nach einem der Ansprüche 2 bis 4, wobei der Ligand unter Tetraphenylporphyrinen, Porphycenen, Porphenen, Porphyrinen, Phthalocyaninen, 1,3-Bis(arylimino)-isoindolinen, Acetylacetonaten, Acetaten, Hydroxiden, Schiffschen Basen, Propanoaten, Butyraten, Benzoaten, Naphthenaten, Stearaten, Porphyrinaten, Porphenaten und Phthalocyanaten ausgewählt ist.

**6.** Verfahren nach einem der Ansprüche 2 bis 4, wobei der Ligand unter Bipyridinen, Terpyridinen, Phenanthrolinen, Dithiocarbamaten, Xanthaten, Salicylaldiminen, Cyclam, Dioxocyclamen, Pyrazoylboraten und Tetraazamacrocyclen ausgewählt ist.

7. Verfahren zur Oxidation von Kohlenwasserstoffen oder partiell oxydierten Kohlenwasserstoffen, das darin besteht, daß die Kohlenwasserstoffe oder partiell oxydierten Kohlenwasserstoffe in Gegenwart eines Katalysators, der ein Übergangsmetallnitrid umfaßt, mit Luft oder Sauerstoff in Kontakt gebracht werden.

8. Verfahren nach Anspruch 7, bei dem die Übergangsmetallnitride unter Nitriden der Gruppen IV bis VIII ausgewählt werden.

9. Verfahren nach Anspruch 7, bei dem die Nitride unter Mangannitrid, Eisennitrid, Chromnitrid und Vanadiumnitrid ausgewählt werden.

10. Verfahren nach einem der Ansprüche 1 bis 9, bei dem die Kohlenwasserstoffe 1 bis etwa 20 Kohlenstoffatome haben.

11. Verfahren nach einem der Ansprüche 1 bis 10, bei dem die Kohlenwasserstoffe Alkane sind.

12. Verfahren nach Anspruch 11, bei dem die Alkane 1 bis etwa 10 Kohlenstoffatome haben.

13. Verfahren nach einem der Ansprüche 1 bis 9, bei dem die Kohlenwasserstoffe oder partiell oxydierten Kohlenwasserstoffe unter Cycloalkanen, alkyl-substituierten Aromaten, Olefinen und partiell oxydierten Derivaten dieser ausgewählt sind.

14. Verfahren nach einem der Ansprüche 1 bis 12, bei dem die Oxidationsprodukte Alkohole, Ketone oder Gemische dieser sind.

15. Verfahren nach einem der Ansprüche 1 bis 13, bei dem die Oxidationsprodukte Alkohole, Ketone, Säuren, Ester oder Gemische dieser sind.

16. Verfahren nach einem der Ansprüche 1 bis 15, wobei die Reaktion in Gegenwart eines organischen Lösungsmittels durchgeführt wird.

17. Verbindung, ausgewählt unter $Mn(Pc)N_3$ und $Cr(Pc)N_3$.

**Revendications**

1. Procédé d'oxydation sélective d'hydrocarbures ou d'hydrocarbures partiellement oxydés, comprenant l'étape consistant à amener lesdits hydrocarbures ou hydrocarbures partiellement oxydés au contact de l'air ou de l'oxygène, en présence d'un catalyseur constitué par un complexe coordonné de métal activé par un azoture ou un nitrure.

2. Procédé selon la revendication 1, où le catalyseur du type complexe coordonné de métal activé par un azoture ou un nitrure répond à la formule :

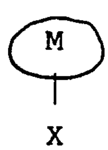

où M est un métal de transition ; X est un azoture ou un nitrure ; et le composant "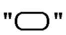" est un ligand.

**3.** Procédé selon la revendication 1, où le catalyseur du type complexe coordonné répond à la formule :

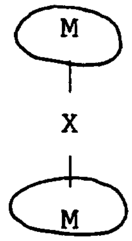

où M est un métal de transition ; X est un azoture ou un nitrure ; et le composant "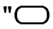" est un ligand.

**4.** Procédé selon la revendication 2 ou 3, où le métal de transition est choisi dans l'ensemble constitué par Ti, V, Cr, Mn, Fe, Co, Nb, Mo, Ru, Rh, W, Os et Ir.

**5.** Procédé selon l'une quelconque des revendications 2 à 4, où le ligand est choisi dans l'ensemble constitué par les tétraphénylporphyrines, les porphycènes, les porphènes, les porphyrines, les phtalocyanines, les 1,3-bis(arylimino)iso-indolines, les acétylacétonates, les acétates, les hydroxydes, les bases de Schiff, les propanoates, les butyrates, les benzoates, les naphténates, les stéarates, les porphyrinates, les porphénates et les phtalocyanates.

**6.** Procédé selon l'une quelconque des revendications 2 à 4, où le ligand est choisi dans l'ensemble constitué par les bipyridines, les terpyridines, les phénanthrolines, les dithiocarbamates, les xanthates, les salicylaldimines, les cyclames, les dioxocyclames, les borates de pyrazoyle et les tétraazamacrocycles.

**7.** Procédé d'oxydation d'hydrocarbures ou d'hydrocarbures partiellement oxydés, comprenant l'étape consistant à amener lesdits hydrocarbures ou hydrocarbures partiellement oxydés au contact de l'air ou de l'oxygène, en présence d'un catalyseur comprenant un nitrure de métal de transition.

**8.** Procédé selon la revendication 7, où les nitrures de métal de transition sont choisis parmi les nitrures des groupes IV à VIII.

**9.** Procédé selon la revendication 7, où les nitrures sont choisis dans l'ensemble constitué par le nitrure de manganèse, le nitrure de fer, le nitrure de chrome et le nitrure de vanadium.

**10.** Procédé selon l'une quelconque des revendications 1 à 9, où les hydrocarbures contiennent de 1 à environ 20 atomes de carbone.

**11.** Procédé selon l'une quelconque des revendications 1 à 10, où les hydrocarbures sont des alcanes.

**12.** Procédé selon la revendication 11, où lesdits alcanes contiennent de 1 à environ 10 atomes de carbone.

**13.** Procédé selon l'une quelconque des revendications 1 à 9, où les hydrocarbures ou hydrocarbures partiellement oxydés sont choisis dans l'ensemble constitué par les cycloalcanes, les composés aromatiques substitués par un groupe alkyle, les oléfines, et des dérivés partiellement oxydés de ceux-ci.

**14.** Procédé selon l'une quelconque des revendications 1 à 12, où les produits de l'oxydation sont des alcools, des cétones, ou des mélanges de ceux-ci.

**15.** Procédé selon l'une quelconque des revendications 1 à 13, où les produits de l'oxydation sont des alcools, des cétones, des acides, des esters, ou des mélanges de ceux-ci.

**16.** Procédé selon l'une quelconque des revendications 1 à 15, où la réaction se déroule en présence d'un solvant organique.

**17.** Composé choisi dans l'ensemble constitué par $Mn(Pc)N_3$ et $Cr(Pc)N_3$.